# EUROPÄISCHE PATENTSCHRIFT

(11) **EP 0 504 163 B1**
(45) Veröffentlichungstag und Bekanntmachung des Hinweises auf die Patenterteilung: **23.03.1994**
(21) Anmeldenummer: 90916724.9
(22) Anmeldetag: 19.11.1990
(51) Int. Cl.: B27H 1/00

(54) **VERFAHREN ZUM DREHRICHTEN VON BRETTERN U.DGL.**
PROCESS FOR THE ROTARY TRUING OF PLANKS AND THE LIKE
PROCEDE DE DRESSAGE SUR GALETS, DE PLANCHES ET ANALOGUE

(30) Priorität: 08.12.1989 DE 3940614
(43) Veröffentlichungstag der Anmeldung: 23.09.1992
(73) Patentinhaber: Gebrüder Linck, Maschinenfabrik "Gatterlinck" GmbH & Co.KG, D-77704 Oberkirch (DE)
(72) Erfinder: GÖNNER, Siegmar, D-7602 Oberkirch (DE)
(74) Vertreter: Katscher, Helmut, Dipl.-Ing.
(86) Internationale Anmeldenummer: EP9001971
(87) Internationale Veröffentlichungsnummer: WO9108878

(56) Entgegenhaltungen:
- WO-A-88/05371
- CH-A- 255 459
- Patent Abstracts of Japan ,vol.10, no.111 (P-451)(2168) 25 April 1986 & JP-A-60 242355 (Minami Kikai) 2 Dec. 1985

## Beschreibung

Die Erfindung betrifft ein Verfahren zum Drehrichten von Brettern u.dgl. um ihre Längsachse mittels eines von den Brettern in Längsrichtung durchlaufenen Brettführungskanals, der einen Drall um die Längsachse aufweist, welcher durch Verstellen von den Brettführungskanal bildenden Teilen veränderbar ist.

Bei einer bekannten Richtbiegevorrichtung (DE-B-32 07 548) werden Bretter und insbesondere in spanloser Schneidtechnik von den Seiten eines angeflachten Holzstammes abgetrennte Bretter und ähnliche flache Holzerzeugnisse, wie Lamellen, die eine Krümmung um eine Querachse aufweisen, zwischen Rollen geradegerichtet.

Vor allem die in der beschriebenen spanlosen Schneidtechnik hergestellten Bretter u.dgl. (auch als Lamellen bezeichnet) weisen aber in vielen Fällen zusätzlich zu der erwähnten Krümmung noch einen Drall auf, d.h. sie sind um ihre Längsachse gedreht oder gewendelt. Diese Verformung schließt in zahlreichen Anwendungsfällen eine automatisierte Weiterverarbeitung der Bretter aus. Der auftretende Drall ist umso stärker, je schräger die das Brett abtrennenden Schneiden gegenüber der Stammlängsrichtung angestellt sind.

Ein bekanntes Verfahren der eingangs genannten Gattung (DE-A-37 01 127) dient dazu, die erzeugten Bretter gerade zu richten. Hierzu durchlaufen die Bretter in ihrer Längsrichtung einen um seine Längsachse gewendelten, d.h. einen Drall aufweisenden Brettführungskanal. Dadurch erhalten die Bretter beim Durchlaufen eine kontinuierliche Verdrehung entgegengesetzt zu ihrer ursprünglichen Drallrichtung. Die Bretter werden dabei so gerichtet, daß sie anschließend eine bleibende gerade Form haben.

Die einzelnen den Brettführungskanal bildenden Teile, beispielsweise Rollenpaare, sind schwenkbar, damit je nach der erforderlichen Richtwirkung ein mehr oder minder starker Drall des Brettführungskanals eingestellt werden kann. Auf diese Weise ist bei dem bekannten Verfahren eine Anpassung an die jeweiligen Anforderungen möglich. Die Einstellung des Brettführungskanals erfolgt nach Erfahrungswerten in erster Linie in Abhängigkeit von dem bei den unbehandelten Brettern vorliegenden Drall. Wenn die Bretter nach dem Durchlaufen des Brettführungskanals noch einen Drall aufweisen, wird die Einstellung des Brettführungskanals geändert, bis ein zufriedenstellendes Ergebnis erreicht wird. Diese Einstellungsarbeiten sind vor allem deshalb sehr aufwendig, weil sich der Ausgangszustand der Bretter häufig ändert und weil die physikalischen und chemischen Eigenschaften des Holzes ebenfalls einen wesentlichen Einfluß haben.

Es ist zwar bekannt (DE-A-36 23 235), die Holzfeuchte als Einflußgröße zur Steuerung eines Bearbeitungsparameters beim spanlosen Abtrennen von Brettern von einem Baumstamm zu verwenden. Dort dient der gemessene Wert der Holzfeuchte dazu, die Anpresskraft eines Druckbalkens zu verstellen, der an der Stelle seitlich auf das Holz drückt, an der die Bretter abgespalten werden. Ein Richtvorgang ist nicht vorgesehen.

Aufgabe der Erfindung ist es daher, ein Verfahren der eingangs genannten Gattung so auszubilden, daß - ausgehend von einer Grundeinstellung - eine selbsttätige Anpassung an die sich ändernden Holzeigenschaften ermöglicht wird.

Diese Aufgabe wird erfindungsgemäß dadurch gelöst, daß die Holzfeuchte des zu richtenden Bretts gemessen wird und die Veränderung des Dralls des Brettführungskanals in Abhängigkeit von der gemessenen Holzfeuchte erfolgt.

Überraschenderweise ist nämlich erstmalig erkannt worden, daß die Holzfeuchte von entscheidendem Einfluß darauf ist, welche Richtverformung des Brettes erforderlich ist, um einen vorgegebenen Drall auszugleichen.

Die Grundeinstellung des Brettführungskanals wird nach dem Drall der unbehandelten, zu richtenden Bretter vorgewählt und ggf. nach einigen Probeläufen so korrigiert, daß ein gerades Brett erzeugt wird. Wenn im weiteren Betrieb Änderungen der Holzfeuchte auftreten, erfolgt in Abhängigkeit davon eine Korrektur der Einstellung des Brettführungskanals, wodurch erreicht wird, daß trotz dieser Änderungen der Holzfeuchte weitgehend gerade Bretter erzielt werden.

Gemäß einer vorteilhaften Ausgestaltung des Erfindungsgedankens ist vorgesehen, daß zusätzlich auch die Holztemperatur des zu richtenden Bretts gemessen wird und die Veränderung des Dralls des Brettführungskanals in Abhängigkeit von der gemssenen Holztemperatur erfolgt.

Es hat sich weiter gezeigt, daß die Einflüsse, die von der Holzfeuchte bzw. der Holztemperatur auf den sich ergebenden Drall des Brettes oder anderen Holzerzeugnisses nicht in zufriedenstellender Weise bereits dadurch erfaßt werden können, daß der Drall des Brettführungskanals empirisch ermittelt wird. Das der Verarbeitung zugeführte Holz hat nämlich in den meisten Fällen keine durchgehend gleiche Holzfeuchte und/oder Holztemperatur. Diese Werte können sich vielmehr in vielen Fällen über die Länge des Holzes ändern. Dies hat seine Ursache darin, daß das Holz vor der Verarbeitung üblicherweise in größeren Stapeln gelagert ist. Die Feuchte und die Temperatur haben in den meisten Fällen im Inneren des Stapels andere Werte als in den Randbereichen. Diese Feststellung gilt einerseits bei Holzstapeln, die im Freien der Witterung ausgesetzt sind; zum anderen gilt diese Feststellung aber auch, wenn derartige Holzstapel in Konditionierkammern vor der Verarbeitung auf höhere Temperatur bzw. höhere Feuchte gebracht werden. Dieses Konditionieren ist für das nachfolgende spanlose Abtrennen von Brettern günstig und wird deshalb bevorzugt angewendet. Gerade dadurch entstehen aber erhebliche Unterschiede der Holzfeuchte und/oder der Holztemperatur, die zu einem unterschiedlichen Drall des erzeugten Brettes führen, der durch einen Richtvorgang ausgeglichen werden muß.

Nur durch die kontinuierliche Erfassung der Holzfeuchte und der Holztemperatur und eine dadurch beeinflußte kontinuierliche Veränderung des Dralls des Brettführungskanals kann der Richtvorgang so gesteuert werden, daß auch unter den genannten erschwerten Bedingungen ein ausreichend gerades Brett erzeugt wird.

Wenn eine Zunahme der Holzfeuchtigkeit und/oder der Holztemperatur festgestellt wird, wird der Drall des Holzführungskanals verringert. Nehmen dagegen diese beiden Werte oder einer dieser Werte ab, so wird der Drall des Holzführungskanals vergrößert.

In Ausgestaltung der Erfindung ist vorgesehen, daß die Veränderung des Dralls des Brettführungskanals zusätzlich in Abhängigkeit von der Vorschubgeschwindigkeit, der Holzart, der Holzbreite und/oder der Brettdicke erfolgt. Die Messung der Holzfeuchte und/oder der Holztemperatur vor dem Einlauf der Bretter in den Brettführungskanal kann vor oder nach dem Austritt der Bretter aus dem Brettführungskanal erfolgen.

Nachfolgend werden Ausführungsbeispiele der Erfindung näher erläutert, die in der Zeichnung dargestellt sind. Es zeigt:
Fig. 1 eine Vorrichtung zum Richten von Brettern in vereinfachter Darstellungsweise in einer Seitenansicht und teilweise im Längsschnitt,
Fig. 2 einen Schnitt längs der Linie II-II in Fig. 1,
Fig. 3 eine vereinfachte Draufsicht auf die Vorrichtung nach den Fig. 1 bis 3,
Fig. 4 in einer Darstellung entsprechend der Fig. 1 eine abgewandelte Ausführungsform der Vorrichtung,
Fig. 5 einen Schnitt längs der Linie V-V in Fig. 4,
Fig. 6 in einer Seitenansicht und teilweise im Längsschnitt eine weitere Ausführungsform einer Vorrichtung nach der Erfindung und
Fig. 7 einen Schnitt längs der Linie VII-VII in Fig. 6.

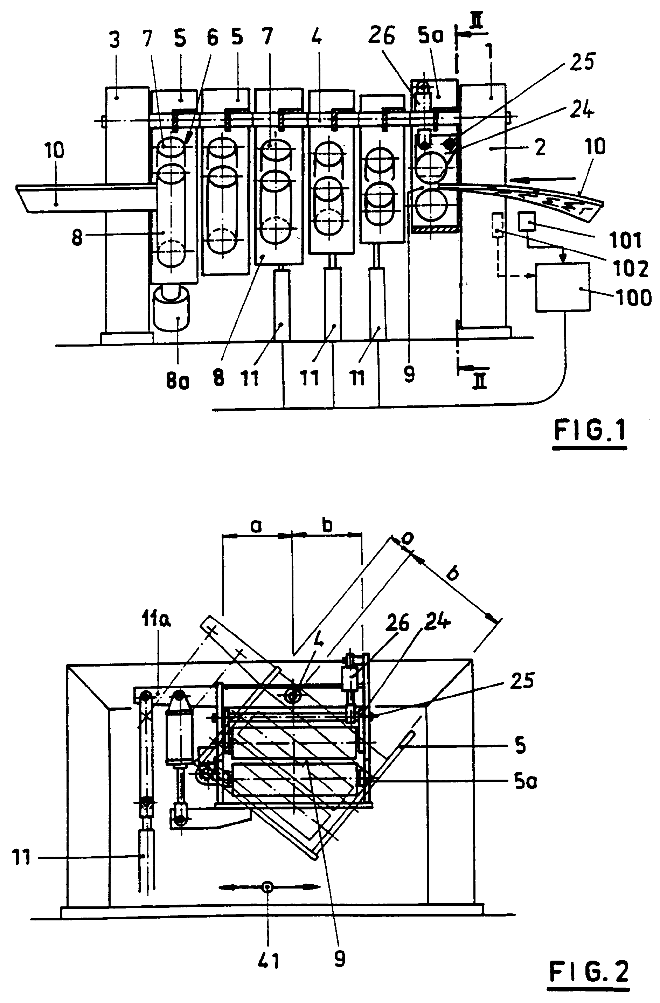

Die in den Fig. 1 bis 3 gezeigte Vorrichtung weist ein Gestell 1 auf, das im wesentlichen aus zwei portalartigen Ständern 2 und 3 am Eingang und am Ausgang der Vorrichtung und aus einer die beiden Ständer 2, 3 verbindenden, sich in Gestellängsrichtung erstreckenden Achse 4 besteht.

Die Achse 4 bildet eine Schwenkachse für mehrere Rollenrahmen 5, die hintereinandergeschaltet sind und jeweils ein Rollenpaar 6 aufweisen, das aus einer oberen Rolle 7 und einer parallel und im Abstand dazu angeordneten unteren Rolle 8 besteht.

In Fig. 2 ist der am Gestelleingang angeordnete Rollenrahmen 5a mit normalen Linien dargestellt. Der am Ausgang angeordnete Rollenrahmen 5 ist ist Fig. 2 in dünnen Linien dargestellt. Man erkennt, daß der Rollenrahmen 5 gegenüber dem Rollenrahmen 5a um die Achse 4 verschwenkt ist. Man erkennt weiter, daß die Schwenkachse 4 beim ersten Rollenrahmen 5a mittig verläuft, d.h. das dort eingetragene Maß a ist gleich dem Maß b. Beim Rollenrahmen 5 verläuft die Schwenkachse 4 außermittig, d.h. das Maß a ist wesentlich kleiner als das Maß b. In entsprechender Weise sind alle Rollenrahmen 5 jeweils gegenüber dem vorhergehenden Rollenrahmen um einen bestimmten Winkelbetrag um die Gestellängsachse verschwenkt oder verschränkt. Dadurch entsteht zwischen den Rollen 7, 8 ein in Gestellängsrichtung verlaufender Brettführungskanal 9 (Fig. 2), der über seine gesamte Länge einen Drall aufweist. Durch diesen Brettführungskanal 9 laufen die zu richtenden Bretter 10 (Fig. 1), die in ihrem Ausgangszustand ebenfalls einen Drall aufweisen, der dem Drall des Brettführungskanals 9 entgegengerichtet ist. Nach dem Durchlaufen des Brettführungskanals 9 treten die Bretter 10 gerade gerichtet aus, wie in Fig. 1 angedeutet ist.

Jeder Rollenrahmen 5 ist mit einem Schwenkantrieb 11 verbunden, der beispielsweise durch einen Druckmittelzylinder gebildet wird, der an einem seitlichen Arm 12 des Rollenrahmens 5 angreift.

Die den einzelnen Rollenrahmen 5 zugeordneten Schwenkantriebe 11 werden von einer Steuerung 100 gemeinsam so gesteuert, daß der Drallwinkel des Brettführungskanals 9 verändert werden kann. Hierzu wird durch einen in Fig. 1 nur schematisch angedeuteten Feuchtesensor 101 die Holzfeuchte des in die Richtvorrichtung einlaufenden Brettes 10 erfaßt; dieser Wert wird der Steuereinrichtung 100 zugeleitet. Ausgehend von einer Grundeinstellung, die bei einer vorgegebenen Holzfeuchte zu gerade gerichteten Brettern führt, erfolgt eine Verstellung des Brettführungskanals 9 in Richtung auf einen stärkeren Drall, sobald der Feuchtesensor 101 eine Verringerung der Holzfeuchte feststellt.

Wie in Fig. 1 mit gestrichelten Linien nur angedeutet ist, kann auch ein Temperatursensor 102 vorgesehen werden, der zusätzlich die Holztemperatur des zu richtenden Bretts erfaßt und diesen Wert ebenfalls der Steuereinrichtung 100 zuführt, so daß die Verstellung des Dralls des Brettführungskanals 9 zusätzlich oder gesondert in Abhängigkeit von der ermittelten Holztemperatur verändert werden kann. Wenn durch den Temperatursensor 102 eine Verringerung festgestellt wird, wird ein größerer Drall des Brettführungskanals 9 eingestellt.

Mindestens eine Rolle 8 im ausgangsseitigen Rollenrahmen 5 ist mit einem Rollenantrieb 8a versehen, der dazu dient, das Brett 10 bis zum Austritt aus der Vorrichtung zu fördern.

Um den Brettführungskanal 9 öffnen zu können, ist jeweils das untere Teil 13 jedes Rollenrahmens 5 bzw. 5a nach unten klappbar. Holzreste oder Bretteile, die sich im Brettführungskanal 9 angesammelt bzw. festgeklemmt haben, können in dieser Weise einfach durch Aufklappen der Rollen 8 entfernt werden, wobei diese Reste entweder von selbst herabfallen oder leicht herausgenommen werden können.

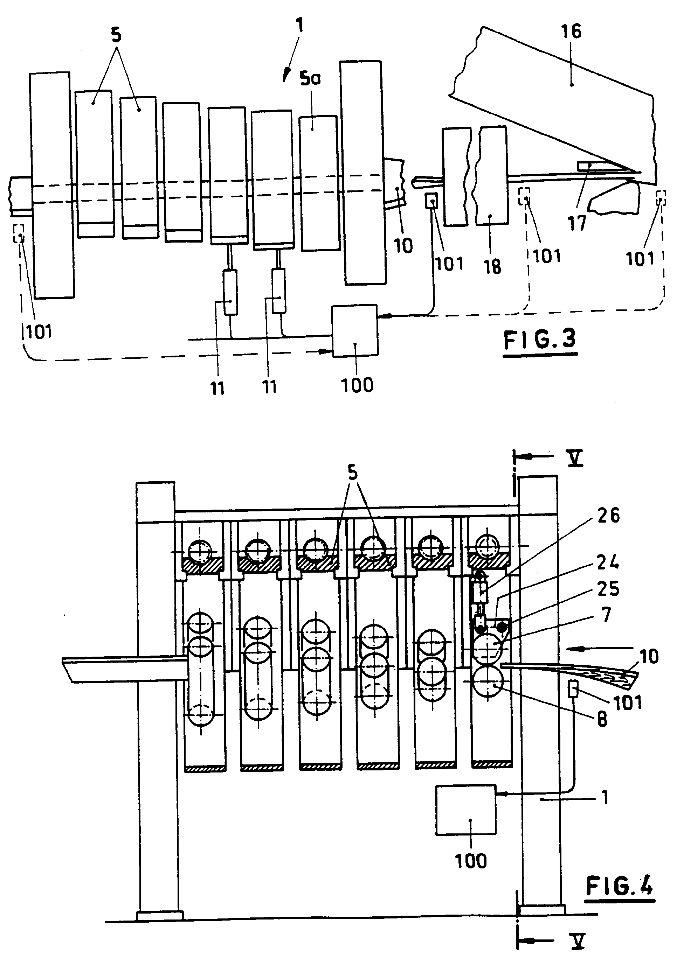

Fig. 3 läßt in einer Draufsicht den seitlichen Versatz der aufeinanderfolgenden Rollenrahmen 5 erkennen. Fig. 3 zeigt außerdem, daß die zu richtenden Bretter 10 beim wichtigsten Anwendungsfall von einem seitlich angeflachten Holzstamm 16 durch ein Messer 17 spanlos abgetrennt werden. Nach Durchlaufen einer in Fig. 3 nur schematisch angedeuteten Richtbiegevorrichtung 18, die eine Krümmung der Bretter 10 beseitigt, treten die Bretter 10 unmittelbar in die Vorrichtung ein, die den Drall der Bretter beseitigt. Die Größe und Richtung dieses Dralls hängt wesentlich von der Schrägstellung der Schneiden 17 ab.

In Fig. 3 ist dargestellt, daß der Feuchtesensor 101 zwischen der Richtbiegevorrichtung 18 und der Drall-Richtbiegevorrichtung angeordnet sein kann. Stattdessen kann der Feuchtesensor 101 aber auch - wie mit gestrichelten Linien in Fig. 3 dargestellt ist - schon am Einlauf der Bretter 10 in die Richtbiegevorrichtung 18 oder auch schon an der Stelle angeordnet sein, an der die Bretter durch die Schneiden 17 spanlos vom Holzstamm 16 abgetrennt werden.

Schließlich ist in Fig. 3 noch angedeutet, daß der Feuchtesensor 101 auch am Ende des Brettführungskanals 9 angeordnet sein kann, um dort die Holzfeuchte zu messen. An diesen Stellen kann auch die Holztemperatur als Einflußgröße für die Steuereinrichtung 100 erfaßt werden.

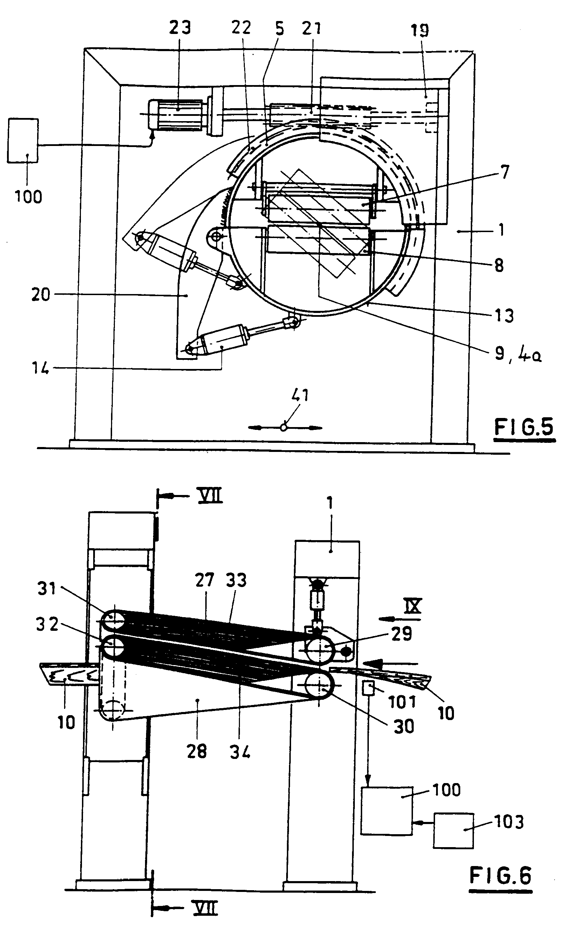

Die in den Fig. 4 und 5 gezeigte abgewandelte Vorrichtung unterscheidet sich von der vorher beschriebenen und in den Fig. 1 bis 3 dargestellten Vorrichtung im wesentlichen nur dadurch, daß die einzelnen hintereinandergeschalteten Rollenrahmen 5 hierbei um eine angenähert mit dem Brettführungskanal 9 zusammenfallende Gestellängsachse schwenkbar sind. Hier bilden die Rollenrahmen 5 jeweils einen Ring, in dem die Rollen 7, 8 gelagert sind. Diese ringförmigen Rollenrahmen 5 sind jeweils in einer Kulissenführung in einem Führungsteil 19 auf einer Kreisbahn geführt, in deren Mittelpunkt die Gestellängsachse liegt.

Auch hierbei ist der Rollenrahmen 5 aufklappbar ausgeführt. Am unteren Rahmenteil 13, in dem die Rolle 8 gelagert ist, greift ein Druckmittelzylinder 14, der an einem mit dem Rollenrahmen 5 verbundenen Arm 20 angebracht ist.

Als Schwenkantrieb ist bei der Ausführung nach den Fig. 4 und 5 ein Schneckentrieb 21 vorgesehen, der ein Schneckensegment 22 am ringförmigen Rollenrahmen 5 mittels eines Stellmotors 23 antreibt.

Auch bei dem in den Fig. 4 und 5 gezeigten Ausführungsbeispiel ist ein Feuchtesensor 101 vorgesehen, dessen Ausgangssignal der Steuereinrichtung 100 zugeführt wird, die in Abhängigkeit von der Holzfeuchte und ggf. von der gemessenen Holztemperatur die Stellmotoren 23 verstellt, um den Drall des Brettführungskanals 9 zu verändern.

Zur Umstellung auf unterschiedliche Brettdicken ist bei den Ausführungsformen nach den Fig. 1 bis 5 vorgesehen, daß in jedem Rollenrahmen 5 bzw. 5a die obere Rolle 7 gegenüber der unteren Rolle 8 verstellbar ist. Hierzu ist die obere Rolle 7 an seitlichen Trägern 24 um eine Schwenkachse 25 schwenkbar. Die Schwenkverstellung erfolgt durch einen Druckmittelzylinder 26.

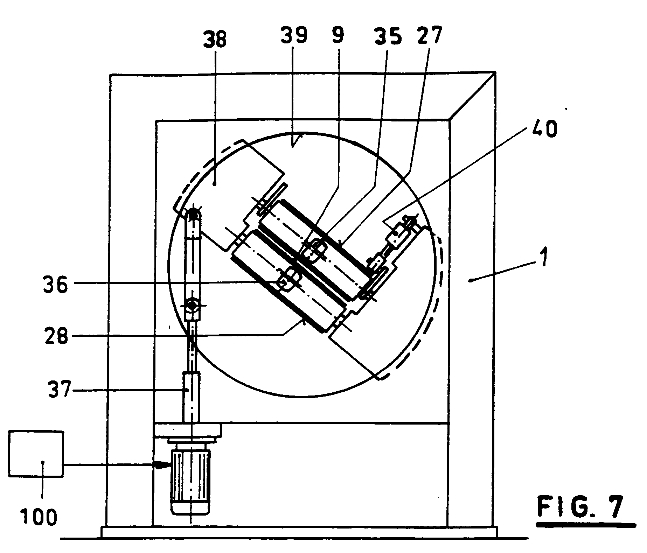

In den Fig. 6 und 7 ist eine weitere Ausführungsform der Vorrichtung gezeigt. Zwei Förderbänder 27, 28 erstrecken sich von einem Eingangsrollenpaar 29, 30 zu einem hiergegen verschränkten Ausgangsrollenpaar 31, 32. Die beiden Förderbänder 27, 28 weisen somit in ihrer Längsrichtung gesehen einen Drall auf; sie bilden zwischen sich den ebenfalls mit einem Drall versehenen Brettführungskanal 9, durch den das zu richtende Brett 10 läuft. Die geschränkten Förderbänder 27, 28 können mit einer Gewebeeinlage versehene Gummibänder oder metallische Gliederbänder bzw. Kettenbänder sein.

Um zu verhindern, daß das durch den Brettführungskanal 9 hindurchtretende Brett 10 im mittleren Bereich der Förderbänder 27, 28 die beiden einander zugekehrten Trumms 33, 34 auseinanderdrückt, können vorzugsweise im mittleren Bereich der Förderbänder 27, 28 an der Rückseite der beiden Trumms 33, 34 jeweils ballige Stützrollen 35, 36 angeordnet sein (Fig. 7). Mindestens eines der beiden Förderbänder 27, 28 ist angetrieben, damit die hindurchtretenden Bretter 10 vollständig aus der Vorrichtung herausgefördert werden.

In Fig. 7 ist dargestellt, daß das Rollenpaar 31, 32 an der Ausgangsseite gegenüber dem Rollenpaar 29, 30 an der Eingangsseite verschwenkbar ist, um den Drallwinkel des gebildeten Brettführungskanals 9 in Anpassung an die jeweiligen Arbeitserfordernisse zu verändern. Hierzu dient ein Schwenkantrieb 37, der einen hier zweiteilig ausgeführten Rollenrahmen auf einer durch einen kreisförmigen Ausschnitt 39 gebildeten Kreisbahn führt.

In den Fig. 6 und 7 ist schematisch dargestellt, daß auch hier die vom Feuchtesensor 101 gelieferten Werte der Holzfeuchte der zugeführten Bretter 10 in der Steuereinrichtung 100 verarbeitet werden, um den Schwenkantrieb 37 zu steuern.

Auch bei dieser Ausführungsform ist an den beiden Rollenpaaren 29, 30 bzw. 31, 32 ein Verstellantrieb 40 vorgesehen, der in der schon beschriebenen Weise eine Verstellung der oberen Rolle 29 bzw. 31 gegenüber der unteren Rolle 30 bzw. 32 zur Anpassung an unterschiedliche Brettdicken ermöglicht.

Auch bei der Ausführungsform nach den Fig. 6 und 7 kann vorgesehen sein, die das untere Förderband 28 tragenden Rollen 30, 32 nach unten abklappbar auszuführen, um den Brettführungskanal 9 zu öffnen, wenn dort angefallene Holzbruchstücke entfernt werden müssen.

Allen dargestellten Ausführungsbeispielen ist gemeinsam, daß eine seitliche Verstellmöglichkeit des gesamten Gestells 1 vorgesehen werden kann, die durch die Pfeile 41 in den Fig. 2 und 5 angedeutet ist.

Bei den Ausführungsformen nach den Fig. 1 bis 5 können die hintereinander angeordneten Rollenpaare 7, 8 auch so ausgerichtet werden, daß ein gekrümmter Verlauf des Brettführungskanals 9 erreicht wird. Auf diese Weise ist es möglich, das Brett 10 auch in seiner Längsrichtung auszurichten, so daß eine gesonderte Richtvorrichtung 18 entfallen kann.

In Fig. 6 ist eine Eingabeeinheit 103 dargestellt, durch die der Steuereinrichtung 10 Steuersignale in Abhängigkeit von weiteren Einflußgrößen eingegeben werden können, um die Verstellung des Brettführungskanals 9 zu beeinflussen, insbesondere die Vorschubgeschwindigkeit, die Holzart, die Holzbreite und die Brettdicke.

## Patentansprüche

1. Verfahren zum Drehrichten von Brettern (10) u.dgl. um ihre Längsachse mittels eines von den Brettern in Längsrichtung durchlaufenen Brettführungskanals (9), der einen Drall um die Längsachse (4) aufweist, welcher durch Verstellen von den Brettführungskanal (9) bildenden-Teilen (6) veränderbar ist, dadurch gekennzeichnet, daß die Holzfeuchte des zu richtenden Bretts gemessen wird und die Veränderung des Dralls des Brettführungskanals (9) in Abhängigkeit von der gemessenen Holzfeuchte erfolgt.

2. Verfahren nach Anspruch 1, dadurch gekennzeichnet, daß zusätzlich die Holztemperatur des zu richtenden Bretts gemessen wird und die Veränderung des Dralls des Brettführungskanals (9) in Abhängigkeit von der gemessenen Holztemperatur erfolgt.

3. Verfahren nach Anspruch 1, dadurch gekennzeichnet, daß die Veränderung des Dralls des Brettführungskanals (9) zusätzlich in Abhängigkeit von der Vorschubgeschwindigkeit, der Holzart, der Holzbreite und/oder der Brettdicke erfolgt.

4. Verfahren nach einem der Ansprüche 1 oder 2, dadurch gekennzeichnet, daß die Messung der Holzfeuchte und/oder der Holztemperatur vor dem Einlauf der Bretter in den Brettführungskanal (9) erfolgt.

5. Verfahren nach Anspruch 1 oder 2, dadurch gekennzeichnet, daß die Messung der Holzfeuchte und/oder der Holztemperatur nach dem Austritt der Bretter aus dem Brettführungskanal (9) erfolgt.

6. Verfahren nach Anspruch 1, dadurch gekennzeichnet, daß der Drall des Holzführungskanals (9) bei einer Zunahme der Holzfeuchte verringert wird.

7. Verfahren nach Anspruch 1, dadurch gekennzeichnet, daß der Drall des Holzführungskanals (9) bei einer Zunahme der Holztemperatur verringert wird.

## Claims

1. Process for the rotary truing of planks (10) and the like around their longitudinal axis by means of a plank guide channel (9) passed through by the planks in a longitudinal direction, which has a twist around the longitudinal axis (4) which can be varied by adjusting the parts (6) forming the plank guide channel (9), characterised in that the timber dampness of the plank to be trued is measured and the modification of the twist in the plank guide channel (9) takes place as a function of the timber dampness measured.

2. Process according to Claim 1, characterised in that the timber temperature of the plank to be trued is additionally measured and the modification of the twist in the plank guide channel (9) takes place as a function of the timber temperature measured.

3. Process according to Claim 1, characterised in that the modification in the twist in the plank guide channel (9) takes place additionally as a function of the feed velocity, the type of timber, the width of the timber and/or the plank thickness.

4. Process according to one of Claims 1 or 2, characterised in that the measurement of the timber dampness and/or the timber temperature takes place before the entry of the planks into the plank guide channel (9).

5. Process according to Claim 1 or 2, characterised in that the measurement of the timber dampness and/or the timber temperature takes place after the exit of the planks from the plank guide channel (9).

6. Process according to Claim 1, characterised in that the twist in the timber guide channel (9) is reduced when the timber dampness increases.

7. Process according to Claim 1, characterised in that the twist in the timber guide channel (9) is reduced when the timber temperature increases.

## Revendications

1. Procédé de dressage tournant de planches (10) et de pièces analogues autour de leur axe longitudinal à l'aide d'un canal guide-planches (9) parcouru par les planches dans la direction longitudinale, lequel canal est soumis à un moment de rotation autour de l'axe longitudinal (4), qui peut être modifié par déplacement de parties (6) formant le canal guide-planches (9), caractérisé en ce qu'on mesure l'humidité du bois de la planche à dresser et on procède à la modification du moment de rotation du canal guide-planches (9) en fonction de l'humidité du bois mesurée.

2. Procédé selon la revendication 1, caractérisé en ce que l'on mesure en outre la température du bois de la planche à dresser et on procède à la modification du moment de rotation du canal guide-planches (9) en fonction de la température du bois mesurée.

3. Procédé selon la revendication 1, caractérisé en ce qu'on procède en outre à la modification du moment de rotation du canal guide-planches (9) en fonction de la vitesse d'avancement, du type de bois, de la largeur du bois et/ou de l'épaisseur de la planche.

4. Procédé selon l'une quelconque des revendications 1 ou 2, caractérisé en ce qu'on procède à la mesure de l'humidité du bois et/ou de la température du bois avant l'introduction des planches dans le canal guide-planches (9).

5. Procédé selon la revendication 1 ou 2, caractérisé en ce qu'on procède à la mesure de l'humidité du bois et/ou de la température du bois après la sortie des planches du canal guide-planches (9).

6. Procédé selon la revendication 1, caractérisé en ce que le moment de rotation du canal guide-planches (9) est réduit lorsque l'humidité du bois augmente.

7. Procédé selon la revendication 1, caractérisé en ce que le moment de rotation du canal guide-planches (9) est réduit lorsque la température du bois augmente.
